Europäisches Patentamt

European Patent Office

Office européen des brevets

(11) Veröffentlichungsnummer: **0 092 024**
**B1**

(19)

# EUROPÄISCHE PATENTSCHRIFT

(12)

(45) Veröffentlichungstag der Patentschrift:
31.07.85

(21) Anmeldenummer: 83100856.0

(22) Anmeldetag: 21.01.81

(60) Veröffentlichungsnummer der früheren Anmeldung
nach Art. 76 EPÜ:

(51) Int. Cl.⁴: **C 01 B 33/18**

(54) **Pyrogen hergestellte Kieselsäure und Verfahren zu ihrer Herstellung.**

(30) Priorität: 25.04.80 DE 3016010

(43) Veröffentlichungstag der Anmeldung:
26.10.83 Patentblatt 83/43

(45) Bekanntmachung des Hinweises auf die Patenterteilung:
31.07.85 Patentblatt 85/31

(84) Benannte Vertragsstaaten:
AT BE CH DE FR GB IT LI LU NL SE

(56) Entgegenhaltungen:
EP - A - 0 015 315
DE - A - 2 620 737
DE - A - 2 909 815
DE - B - 2 123 233
US - A - 3 772 427

(73) Patentinhaber: Degussa Aktiengesellschaft,
Weissfrauenstrasse 9, D-6000 Frankfurt am Main 1 (DE)

(72) Erfinder: Schwarz, Rudolf, Dr., Taunnusstrasse 2,
D-8755 Alzenau (DE)
Erfinder: Kleinschmit, Peter, Dr., Wildaustrasse 19,
D-6450 Hanau 9 (DE)

## Beschreibung

Die Herstellung pyrogener hochoberflächiger Kieselsäuren durch Hochtemperaturhydrolyse von Siliciumhalogeniden, insbesondere von Siliciumtetrachlorid in einer Wasserstoffflamme, ist nicht neu. Seit Jahrzehnten werden nach diesem Verfahren große Mengen sehr reiner hochdisperser Kieselsäuren hergestellt, die als Verdickungsmittel in der Lack- und Farbenchemie, bei der Herstellung pasteuser oder salbenartiger Massen, z. B. bei der Zahnpastenbereitung und in der pharmazeutischen Industrie eine sehr große Verbreitung gefunden haben.

Es ist bekannt, daß der Grad der mit der pyrogenen Kieselsäure erzielbaren Verdickung von vielerlei Faktoren abhängig ist. Insbesondere bestehen Anhängigkeiten zur Art des Systems, das verdickt werden soll, zur Kieselsäuretype und ihrer angewendeten Konzentration, zum pH-Wert des Systems und zum Grad der Dispergierung, der mit einem bestimmten Dispergierungsaufwand erreicht wurde.

Dem Einfluß der Kieselsäuretype kommt eine besonders große Bedeutung zu. Die Verdickung eines Systems ist nämlich um so größer, je höher die Aktivität der verwendeten Kieselsäuretype ist. Als Maß für die Aktivität kann man vereinfachend die äußere spezifische Oberfläche einer Kieselsäure ansehen. Aus diesem Grund sind auch die pyrogenen Kieselsäuretypen nach der Größe der spezifischen Oberfläche geordnet. Es trifft aber nicht zu, daß bei einem gegebenen +ispergierungsaufwand die Kieselsäuretype mit der höchsten spezifischen Oberfläche auch die größte Verdickungswirkung ausübt. Vielmehr ist es im allgemeinen so, daß die Verdickungswirkung mit steigender spezifischer Oberfläche der Kieselsäure zunächst bis zu einem Maximum zunimmt und sich bei weiter steigender Oberfläche wieder vermindert. So hat zum Beispiel eine handelsübliche pyrogene Kieselsäure in einem ungesättigten Polyester-Testharz bei gegebener Konzentration folgende Verdickungswirkungen in Abhängigkeit von der spezifischen Oberfläche:

| Spez. Oberfläche $m^2/g$ | Viskosität mpas |
|---|---|
| 130 | 2 000 |
| 200 | 2 900 |
| 300 | 3 500 |
| 380 | 3 000 |

Wie man sieht, kommt man bei einer gegebenen Konzentration an Kieselsäure über eine Viskosität von ca. 3500 mpas nicht hinaus.

Es gibt zwar zahlreiche betriebstechnische Möglichkeiten, bei der Herstellung pyrogener Kieselsäuren zu noch höheren spezifischen Oberflächen zu gelangen. Sie alle führen aber, wie gezeigt wurde, bei gleichen mengenmäßigen Verhältnissen nicht zu einer Verbesserung der verdickenden Wirkung der pyrogenen Kieselsäure. Andererseits sind die betriebstechnischen Möglichkeiten, die verdickende Wirkung von pyrogener Kieselsäure bei gegebener spezifischer Oberfläche zu verbessern, sehr eng begrenzt oder erfordern einen unvertretbaren hohen Aufwand.

Gegenstand der Erfindung ist ein Verfahren zur pyrogenen Herstellung von Kieselsäure, welches dadurch gekennzeichnet ist, daß man als Rohstoff ein Gemisch aus Siliziumtetrachlorid und/oder Methyltrichlorsilan und Hexamethyldisiloxan verwendet.

Auf diesem Wege konnten Produkte erhalten werden, die eine bisher nicht gekannte verdickende Wirkung auf die verschiedensten flüssigen Systeme ausüben.

Die folgenden Beispiele sollen den Erfindungsgegenstand näher erläutern und beschreiben:

### Beispiel 1
### (Vergleichsbeispiel)

In einem Verdampfer werden 3,4 Liter Siliciumtetrachlorid pro Stunde, das sind 5,03 kg, verdampft und mit 4,3 $m^3$ auf 80°C erwärmte Luft vermischt. Das Silan-/Luftgemisch wird sodann in das Schaftende des Brenners, gemäß US-PS 3 086 851 tangential eingeleitet und dort mit 1,76 $m^3/h$ Wasserstoff, der durch einen zweiten Stutzen einströmt, homogen vermischt. Die sternförmigen Einbauten des Brennerschaftes bewirken eine Laminierung der Gasgemische, die an der Brenneröffnung mit einer Geschwindigkeit von 23,8 m/sec austritt und dort nach der Zündung abbrennt. Der Brenneraustritt hat einen Durchmesser von 10 mm. Zur Stabilisierung der Flamme und zur Verhinderung von Ansätzen am Brennerende läßt man einen die Brennermündung umgebenden Ringspalt 0,35 $m^3/h$ Wasserstoff austreten und als Flammenmantel ebenso abbrennen. Die heißen Reaktionsprodukte der Flammenzone werden in eine Kühlkammer eingesaugt und nach der Abkühlung auf <150°C pneumatisch einer Filtrationsanlage zugeführt. In der Filteranlage trennt man die erhaltene pyrogene Kieselsäure von den gasförmigen Reaktionsprodukten Sauerstoff, Stickstoff, Wasserdampf und Chlorwasserstoff. Die noch etwa 1% Chlor enthaltende Kieselsäure wird anschließend in einer auf 600°C geheizten Reaktionsstrecke mit Hilfe eines Wasserdampf-/Luftgemisches nachentsäuert und schließlich in einem Bunker abgesetzt. Die Ausbeute pro Stunde beträgt 1,78 kg $SiO_2$.

Die spezifische Oberfläche der· erhaltenen Kieselsäure, bestimmt nach BET, beträgt 215 m²/g. Mit diesem Produkt läßt sich ein ungesättigtes Polyesterharz in einem Test, wie er weiter unten beschrieben ist, auf eine Viskosität von 2980 mpas bringen. Mit dem gleichen Produkt erreicht man in einer Zahnpasta-Testrezeptur, die weiter unten ist, eine Verdickung von 2200 mpas.

### Beispiel 2
### (Vergleichsbeispiel)

In gleicher Weise wie im Beispiel 1 werden 0,546 Liter Hexamethyldisiloxan = 0,752 kg pro Stunde verdampft und mit 7,11 m³/h auf 120°C vorerwärmter Luft vermischt. Das Siloxan-/Luftgemisch überführt man in den Brenner gemäß US-PS 3 086 851 und läßt es an der Brenneröffnung mit einer Geschwindigkeit von ca. 25,5 m/sec ausströmen und abbrennen. Die Reaktionsflamme ist wie im Beispiel 1 mit einer reinen Wasserstoffflamme von 0,35 m³ $H_2$/h eingemantelt. Nach dem Abkühlen der Reaktionsprodukte aus der Flamme in der Kühlzone auf < 150°C wird die erhaltene Kieselsäure von der gasförmigen Reaktionsmischung aus Stickstoff, Sauerstoff, Wasserdampf und Kohlendioxid durch eine Filtration oder mit Hilfe eines Zyklons abgetrennt.

Es werden pro Stunde 0,55 kg pyrogene Kieselsäure erhalten. Die spezifische Oberfläche nach BET der Kieselsäure beträgt 159 m²/g. Die Viskosität eines nach der Prüfvorschrift gemäß Beispiel 1 verdickten ungesättigten Polyesterharzes liegt bei 1575 mpas.

### Beispiel 3

Zum Nachweis, daß sich mit Gemischen aus Siliciumtetrachlorid und/oder Methyltrichlorsilanen und Hexamethyldisiloxan pyrogene Produkte mit gegenüber Beispiel 1 verbesserten Eigenschaften herstellen lassen, wird ein Gemisch von 0,41 Liter/h Hexamethyldisiloxan (0,565 kg) und 1,7 Liter/h Siliciumtetrachlorid (2,516 kg) verdampft, mit 9,25 Nm³ vorerwärmter Luft gemischt und in den Brenner gleichzeitig mit 1,612 m³/h Wasserstoff eingeleitet. (Eine Alternative stellt folgende Mischung dar, die zu gleichen Ergebnissen führt: 0,565 kg/h Hexamethyldisiloxan, 1,89 kg/h Siliciumtetrachlorid, 0,563 kg/h Methyltrichlorsilan. Die Menge Wasserstoff und vorerwärmte Luft werden wie oben verwendet.) Die Gasmischung verläßt den Brenner mit einer Geschwindigkeit von 39,9 m/sec und brennt nach der Zündung am Austritt ab. Die Mantelwasserstoffmenge beträgt 0,350 m³/h. Die Kieselsäure wird wieder, wie im Beispiel 1, zunächst nach Abkühlung von den gasförmigen Reaktionsprodukten getrennt und dann mit Luft/Wasserdampf bei 600°C vom restlichen Chlor befreit. Es werden pro Stunde 1,3 kg pyrogener Kieselsäure erhalten, die zu 68,1% aus dem Siliciumtetrachlorid und zu 31,9% aus dem Hexamethyldisiloxan stammen. Die Viskosität von Poly

esterharz gemäß der Prüfvorschrift von 3690 mpas ist immer noch deutlich höher als die der Kieselsäure gemäß Beispiel 1. Desgleichen ist die Viskosität der Zahnpastengrundmasse mit 2914 mpas höher als die der Kieselsäure gemäß Beispiel 1.

### Prüfung des Verdickungsverhaltens
### von pyrogener Kieselsäure
### bei Dissolverdispergierung

#### 1. Grundlage

Maßstab des Verdickungsverhaltens ist die Viskosität eines mit pyrogener Kieselsäure vermischten Polyesterharzes.

Die Einheit der dynamischen Viskosität ist die Pascalsekunde (Pas). Eine Millipascalsekunde (mPas) entspricht der bisher gebräuchlichen Einheit Centipoise (cP). Die Bestimmung der Viskosität erfolgt mit einem Rotationsviskosimeter nach DIN 53 214.

#### 2. Geräte und Reagenzien

Rotovisko RV 1-3 oder Nachfolgetypen (Firma Haake, Karlsruhe) mit Wasserthermostat, Prüfkörper MV 2 und Meßkopf 50.

Dissolver, Ø der Dispergierscheibe 5 cm
Plastikbecher, äußerer Durchmesser 8,4 cm
Ludopal P6 (BASF, mit einer Viskosität von 1100 ± 100 mPas)
Monostyrollösung (100 g Monostyrol und 0,4 g Paraffin)
Tafelparaffin 50/52°C, Ölgehalt 1—1,5%, Firma Jung, Atlantic Refining GmbH, Hamburg

#### 3. Durchführung

In 142,5 Gew.-Teile Ludopal® P6 werden 7,5 Gew.-Teile pyrogene Kieselsäure in einem Plastikbecher eingewogen (mit dem Spatel von Hand vermischt) und mit einem Dissolver fünf Minuten lang bei 3000 Upm dispergiert (Dissolverscheibe ca. 1 mm vom Boden des Plastikbechers entfernt).

60 g der erhaltenen Paste werden mit 63 g Polyesterharz und 27 g Monostyrollösung versetzt, mit einem Spatel gut verrührt und in dem genannten Plastikbecher mit dem Dissolver drei Minuten bei 1500 Upm dispergiert.

Um Verluste an Styrol zu vermeiden, wird der Becher während der Dispergierung mit einer Plastikdecke abgedeckt. Zur Entfernung von eingeschlossenen Luftbläschen wird die Probe kurzzeitig evakuiert.

Nach einer Standzeit von 1 Stunde und 45 Minuten wird die Probe in den Meßbecher eingefüllt. Der Meßbecher wird in den Temperierkopf eingeschraubt und bei 22°C temperiert. Nach 15 Minuten Standzeit erfolgt die Viskositätsmes

sung bei einem Schergefälle von $D = 2,72\,s^{-1}$. Der Zeiger am Anzeigeinstrument wird nach 30 Sekunden abgelesen. Nach dieser Zeit ist ein annähernd konstanter Wert erreicht.

### 4. Auswertung

Aus der vorgelegten Geschwindigkeit, dem Zeigerausschlag und der Eichkonstante des verwendeten Drehkörpers erhält man die Viskosität nach der Gleichung:

$\eta = U \cdot S \cdot K\,(\text{mPas})$

U — Geschwindigkeitsstufe des Rotovisko (bei RVI = 162)

S — angezeigte Skalenteile

K — Eichkonstante des verwendeten Drehkörpers

### Testrezeptur für Zahnpasten

Zusammensetzung der Phosphatgrundmasse:

| | |
|---|---|
| Wasser | 35,05 |
| Dehydazol A 400 P | 0,80 |
| Solbrol M-Na | 0,15 |
| Glyzerin | 12,00 |
| Sorbitol | 15,00 |
| Dicalziumphosphat-Dihydrat (L) | 34,00 |
| | 97,00 |

In 97 g dieses Vorkonzentrates wurden 3 g pyrogene Kieselsäure mit einem Spatel eingearbeitet und anschließend 3 × auf einem Dreiwalzenstuhl homogenisiert. Anschließend wurde die eingeschlossene Luft im Exsiccator entfernt. Von der so hergestellten Paste wurde nach 1. Tag die Viskosität mit dem Rotovisko System PK Nr. 8012 Geschwindigkeit 27 bestimmt.

**Patentanspruch**

Verfahren zur pyrogenen Herstellung von Kieselsäure, dadurch gekennzeichnet, daß man als Rohstoff ein Gemisch aus Siliciumtetrachlorid und/oder Methyltrichlorsilan und Hexamethyldisiloxan verwendet.

**Claim**

Process for the pyrogenic production of silica, characterised in that a mixture of silicon tetrachloride and/or methyl trichlorsilane and hexamethyldisiloxane is used as the raw material.

**Revendication**

Procédé pour la préparation de silicie par voie pyrogénique, caractérisé en ce que comme matière première, on utilise un mélange de tétrachlorure de silicium et/ou méthyltrichlorosilane et d'hexaméthyldisiloxane.